# EUROPEAN PATENT SPECIFICATION

(11) **EP 0 347 779 B1**
(45) Date of publication and mention of the grant of the patent: **04.05.1994**
(21) Application number: 89111020.7
(22) Date of filing: 17.06.1989
(51) Int. Cl.: A61M 15/00

(54) **A device for dispensing metered amounts of aerosol for inhalation**
Einrichtung zum Abgeben von abgemessenen Mengen Aerosol zur Inhalation
Dispositif pour administrer des quantités mesurées d'aérosol pour inhalation

(30) Priority: 22.06.1988 IT 2106588
(43) Date of publication of application: 27.12.1989
(73) Proprietor: CHIESI FARMACEUTICI S.p.A., I-43100 Parma (IT)
(72) Inventor: Chiesi, Paolo, I-43100 Parma (IT)
(74) Representative: Minoja, Fabrizio

(56) References cited:
- EP-A- 0 009 667
- DE-A- 1 467 752
- GB-A- 2 110 543

## Description

Inhalation is the preferred way of administering drugs that are directed to the deepest parts of the respiratory tree because it markedly reduces the dosage in comparison with the oral way; it eliminates almost completely the systemic side effects; it allows the therapeutical action to be rapidly established.

The pressure aerosols, due to their ease of handling and to the feature that they allow the active ingredient to be rapidly and selectively administered, have met a great favour and are widely used both in the maintenance therapy of obstructive chronic respiratory diseases and in the treatment of acute fits of asthma.

Their apparent simplicity notwithstanding, the usual metered aerosols are difficult to use properly, and many reports from the scientific literature point out that the majority of patients employ the apparatus in an improper way, either because they are not able to time the delivery in synchronism with breathing-in and do not inhale at the right time, or because they do not keep a suitable inspiration flow, or because they do not inhale deeply enough, or due to different reasons.

This problem is even more prominent with particular subject, such as the children, elderly people and patients having reduced capabilities either from the point of view of respiration or from a manual point of view.

Even when the apparatus is used in the right way, the availability of an inhaled drug to the air pathways depends in a large measure on the dimensions of the aerosol droplets, that in turn depend on the formulation and the evaporation time of the solvent. It is well supported by documentary evidence that even in the most favourable conditions only 10% of the dispensed dosis of a metered aerosol reaches the air pathways: a similar percent is expired or deposited in the dispenser, while about 80%, due to the impact of high speed particles of aerosol, are deposited onto the mouth cavity and the pharynx, then being swallowed up and resorbed at a systemic level.

The inhaled portion of the drug, however, is usually enough to obtain the therapeutical effect.

Nevertheless, if the apparatus is not used in a proper way, the amount of drug reaching the action site at the lung level is further reduced and the therapeutical response is compromised. An excess deposition of active principle in the mouth-pharyinx cavity can moreover give rise to undesirable effects both at systemic level as a consequence of the resorption of the drug and at a local level, such as it is the case with corticosteroids, that can give rise to mouth candidosis.

In a search to obviate the drawbacks that are connected with the use of metered aerosols, there have been developed in recent years expansion chambers.

Said expansion chambers can be divided, according to their dimension features, in SPACERS and RESERVOIR BAGS or, more simply, RESERVOIRS.

The "spacers" are essentially expansion tubes that are inserted between the dispenser and the mouth to the purpose of increasing the proportion of drug that is deposited at a lung level, by acting on two factors: the dimension of the aerosol droplets and their impact in the mouth-pharyinx cavity.

The latency period between spray delivery and inhalation permits in fact a quick evaporation of the propellant with a reduction of the size of the particles before they enter the respiratory tree, thus helping them to better penetrate till the lower air pathways.

At the same time, the distance between dispenser and mouth thus obtained causes a slow-down of the particle speed, thus reducing the proportion of active principle that is lost due to the immediate impact against the walls of the oral cavity.

Finally a lower amount of propellants is inspired, with the consequence of a better compliance by the patient and a lower risk due to their possible toxicity.

The expansion chambers that are conventionally defined as spacers, share the features of a cylindrical shape and a rather low volume (70-100 ml).

Various spacer models are known:
- simple devices that are in practice comparable with a cylindrical tube with mouth-piece, in which the dispenser is inserted in a perpendicular position (Fisons EP 89070, Huhtamaki WO 8502778); or in a parallel position (Newhouse EP 9667) relative to said tube;
- apparatuses having a cylindrical shape as well in which the dispenser is in a transversal position relative to the spacer, said spacer being possibly telescopically extensible at the moment of use (Draco EP 15247 and EP 74937);
- spacers comprising a first expansion chamber with a cylindrical shape, followed by a second one having a larger cross-section (Vortran Corp. PCT 2345/85, Hughes N. US 4690332);
- further similar models that simply have the advantage that they can be disassembled after use and employed as a container for the dispenser (American Cyanamid US 3994421, US 3897779, US 3809294 and Rorer US 4637528).

EP-A- 0009667 describes a further spacer model, wherein a one way valve is provided in the mouthpiece.

The use of this type of device has rapidly spread with the aim of helping those patients that are not able to use in a proper way the usual metered aerosols or that meet difficulties in understanding their rather complex instructions.

The reports from the scientific literature do nevertheless agree that the application of small spacers does not produce appreciable improvements at a clinical level.

For this reasons, the research in this field has been addressed to providing large volume expansion chambers that are called "reservoirs".

The reservoirs, beside including the advantages of spacers, should also contribute in resolving the great problem of non-coordination between the dispensing maneuver and the inspiration because they permit the inspiration to be delayed of a few seconds relative to dispensing, thus helping also the patients having serious breathing difficulties.

The reservoirs share the characteristic of a great volume (about 700-1250 ml), while they exhibit various shapes, materials and costruction characteristics. The main reservoir types that are described in literature are as follows:
- rigid containers, with a round or pear-shape in which the usual spray can be inserted either in a position contiguous to the inhaling mouth-piece (Langaker K.E. SE 8200322) or in a diametrically opposite position (Draco SE 76012448);
- bellows - like chambers, that should help the inspiration maneuvre of the active principle, having the shape of collapsible bag (Key EP 50654) or of a cylindrical bag, flexible only along the side surface (Key EP 108145, Johns Hopkins Univ. EP 251443);
- containers having a regular cylindrical shape, with a mouth-piece in a side position relative to the port for the connection to the can (Trutek US 4534343), or in an opposite position;
- devices that can be folded back after use and comprising a set of jointed telescopic beakers made of a transparent plastic material (Southampton Univ. GB 2110543), or a flexible bag provided within a rigid, telescopically retractible cylindrical container (Deshpaude GB 2182249).

The side and volume of these reservoirs are however such that in practice they are only limited to a domestic use.

DE-A- 1,467,752 discloses a device for dispensing an alkaloid substitute of the nicotine in order to decrease the consumption of cigarettes in smokers. Such a device comprises two coaxial tubes which slides within one another and connectable to an aerosol dispensing flask, which can be housed in the device.

We have now developed a new type of expansion chamber the size of which is comparable with that of spacers, but providing the effectiveness and way of use of the reservoirs.

The object of the invention is to overcome the drawbacks of either apparatuses.

The subject device of the present invention comprises in fact the following features:
1) the volume of the chamber is 4-8 times greater than that of an usual spacer, with the consequence of a remarkable increase of the efficiency in slowing-down and evaporating the aerosol microdroplets;
2) it can be used as a reservoir by first dispensing and then inhaling the product, with no need of synchronising the two operations;
3) a one-way valve is provided at the mouth-piece, the function of said valve being both to prevent the aerosol spray that has been delivered into the chamber from exiting before the inhalation, and the patient from expiring into the apparatus;
4) the volume of the internal chamber can be adapted, thus allowing a diversified use according to the needs of the patients;
5) various kinds of dispensers can be inserted, whereby the apparatus can be used with different active substances;
6) the product can be housed inside the apparatus for an easier transportation;
7) its size is very limited, even though it has an appreciable volume of the expansion chamber, thereby making it easy to carry it even in limited spaces (pocket, bags, and so on), thereby overcoming the problem of a strictly domestic use;
8) as a consequence of the previous point, a higher effectiveness/dimensions ratio is achieved;
9) its use is very simple, thereby making it easily handled also by children.

The invention will now be described with reference to a merely illustrative, non-limiting embodiment thereof, said embodiment being illustrated in the enclosed drawing, in which:
- figure 1 is a longitudinal section, in a diagrammatic representation, of the device according to the invention for dispensing metered aerosols to be inhaled; in the upper half of the figure, the internal tube is shown at the maximum extension position relative to the outer tube, while in the lower half the internal tube is in a completely receded position relative to the outer tube, corresponding to the stand-by condition of the apparatus;
- figure 2 is a section along line A-A of figure 1;
- figure 3 is a cross section of the mouth-piece of the apparatus for dispensing metered aerosols to be inserted into the rear opening of the internal tube;
- figure 4 is a view of the mouth-piece of the reservoir in the direction of arrow B of figure 1.

The apparatus essentially comprises two coaxial tubes 1, 2 that are freely slidable within one another and separable. Said tubes delimit an internal chamber 3 the volume of which can be varied from about 200 ml to about 600 ml, depending on the degree of extension of the internal tube (1) relative to the outer one (2). The lenght of the chamber (3) may range from 100 to 300 mm and the maximum diameter from 50 to 80 mm.

The outer tube (2) (o.d.: 59.5 mm) has an open end 4, in which the internal tube can be inserted, while the opposite end 5 is tapered with a circular cross section (o.d.:33 mm). In end 5 four spokes 6 are provided in an orthogonal arrangement, said spokes stopping the four tabs 7 of a four-cuspid valve 8 against its opening towards the interior.

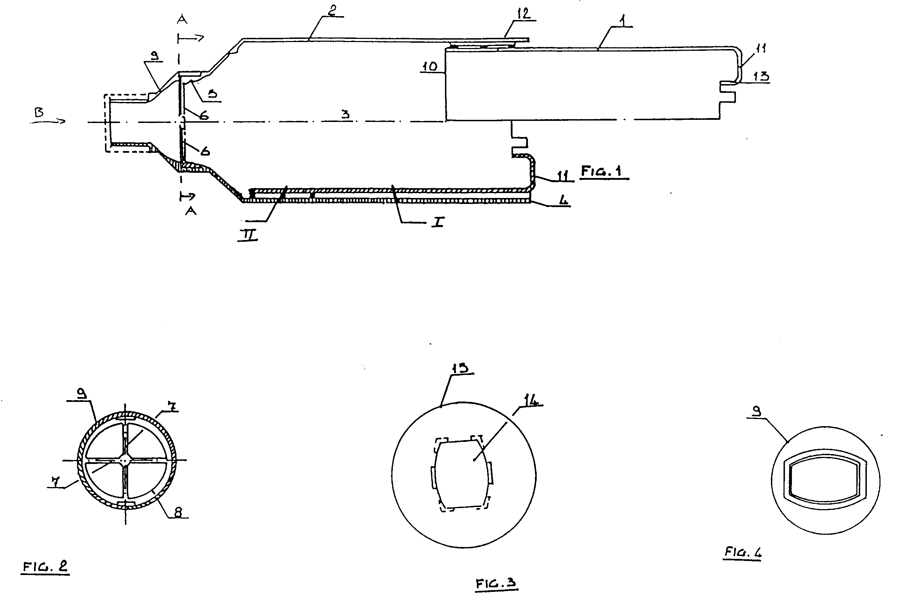

On the tapered end 5 (figure 2) the valve 8 is seated, whose characteristics are particularly important for the optimal operation of the apparatus as a whole.

Its purpose is to prevent both the leakage of the aerosol spray delivered into the chamber, before the inhalation, and the patient's expiration into the apparatus itself.

The cross section of the same configuration shown in figure 2 moreover allows, during inhalation, the aperture of an hole wide enough not to hinder the passage of the material to be inhaled also for a patient having respiratory difficulties.

Onto valve 8 the mouth-piece 9 of the reservoir is then assembled, said mouth-piece 9 having a final cross section like the final cross section of the dispensing mouth-piece 15 of the can (figure 3).

The internal tube 1 (o.d.: 51.5 mm) also includes an open end 10 that delimits the volume of the chamber, and an opposite end 11 provided with an opening 13 the cross section of which is slightly larger then that of the end opening 14 of the dispensing mouth-piece 15 (figure 3). Said opening 13 is also provided on the inner side with a set of lamellae (the number of said lamellae being four or six) that allow various kinds of mouth-pieces to be forced in.

The internal tube 1 slides within the outer tube 2, a polyethylene gasket 12 being provided between the two tubes, said gasket 12 being fixed outside at the open end 10. Said gasket provides the seal of the apparatus against a leakage of the aerosol material and for the solidity of the assembly in the opened position.

Tubes 1, 2, as above-mentioned, can be separated; that is, the internal tube 1 can be completely removed from the outer tube 2. Said feature has a double pourpose: first it allows the spray can with mouth-piece to be housed inside the chamber 3, thus making it easier to be transported; secondly it permits a thorough cleaning of chamber 3.

The outer tube 2 carries two graphic symbols embossed on the outer surface: the first one, at the mouth-piece 9, shows the patient the end of the apparatus from which he should inhale; the second one is an arrow at the opposite end of tube 2 and on the same axis, directed towards the inner tube 1.

Said inner tube, in turn, carries two arrows embossed on the outer surface and on the same axis spaced about 37 mm from each other and directed towards the outer tube 2. Near said latter arrow the symbols I (near the proximal arrow relative to the bottom of the tube) and II (distal arrow), are embossed, said marks being provided to show the two extention positions recommended for use by children and adults, respectively.

An ideogram is finally embossed on the inner tube, at the bottom and coaxially relative to the above-mentioned arrows, said ideogram indicating the side of the apparatus in which the dispensing mouth-piece 15 of the can is to be introduced.

The apparatus has a double function: first it causes the aerosol cloud delivered into the chamber to expand, thus slowing down the micro-droplets and enhancing the evaporation of the propellant therefrom, thereby reducing the average aerodynamic dimensions of the aerosol particles at the moment of inhalation and increasing the proportion of active substance having a size such as to allow it to reach the action site deep into the respiratory tree.

Secondly, the way of use of the apparatus, where the substance is first dispensed into chamber 3 and then inhaled from the opposite side through one-way valve 8 (that opens towards the patient at the moment of inspiration but not during expiration), allows it to be used by that remarkable proportion of patients having a chronic obstructive broncho-pulmonar illness that are not in a measure of properly using a normal MDI either because they cannot coordinate the delivery with the beginning of the respiratory act, or because they oppose an expiratory reflex upon the violent entry of the aerosol jet into the mouth.

The way of use of the device is very simple.

As already stated, the apparatus can be operated in connection with various types of metered aerosols.

Cap 15 is removed from the mouth-piece of the dispenser containing the prescribed product, then placing it onto the mouth-piece 9 of the expansion chamber 3. The internal tube 1 is then completely extracted and the product is housed into it; the internal tube is then completely inserted again into the outer tube 2. The assembly is now in a standby position, well closed, easily transportable even within a limited space.

At the moment of use, the assembly is removed from the bag, the cap is removed from mouth-piece 9, the internal tube 1 is completely extracted and the mouth-piece-carrying can is removed therefrom, the inner tube 1 is inserted again into the outer tube 2 until the opposite arrows on the two tubes are aligned (whereby two different positions can be selected by aligning the arrow on the outer tube with the first (position 1: children) or the second (position 2:adults) arrow of the inner tube, said position corresponding to a volume of chamber 3 of 290 ml or 380 ml, respectively).

The product carrying the mouth-piece is then inserted in an upside-down position into the bottom opening of the inner tube 1, pressing it until it is solidly blocked.

The mouth-piece 9 of the receiver is then taken between the lips, followed by completely expiring, dispensing the spray by pressing once on the bottom of the can and slowly breathing-in. The breath is held for at least 7 seconds before expiring again. Should it be necessary to administer a second dosis, the above-mentioned procedure is repeated after allowing a time period of at least 30 seconds to elapse.

Finally, the dispensing mouth-piece 15 is removed from the reservoir, the reservoir is opened again by removing the internal tube 1, the mouth-piece-carrying can is introduced into it and the assembly is closed again. The cap is applied again to the mouth-piece 9 of the reservoir and the assembly is finally put away.

Comparison tests with metered aerosols of a conventional type, provided or not with the expansion chamber of the present invention, have proved that this device allows the amount of active substance that reaches the lower respiratory pathways, where it develops its therapeutical action, to be improved.

## Claims

1. A device for the administration of drugs in the form of metered aerosols comprising two coaxial tubes (1, 2) freely slidable within one another, said tubes delimiting an internal expansion chamber (3) the volume of which can be varied according to the amount of extension of the internal tube (1) relative to the external tube (2), said external tube (2) having an open end (4) for inserting the internal tube (1) and an opposite end (5), and the internal tube (1) also having an open end (10) inserted in the external tube (2) and an opposite end (11), connectable with an aerosol dispenser, said opposite end (5) of the external tube (2) being tapered and a one-way valve (8) being housed therein, on which valve a mouth piece (9) is mounted and the opening of mouth-piece (15) of the dispenser, the tubes (1, 2) of the expansion chamber (3) and the opening of the mouth-piece (9) of the latter being on the same axis, characterised in that said end (11) of the internal tube (1) having an opening (13) provided on the inner side with a set of lamellae on which opening various kinds of mouth-pieces (15) of conventional metered aerosols dispensers can be stuck.

2. A device according to claim 1, characterized in that the volume of the expansion chamber (3) can be varied between about 200 and 600 ml, the total length can be varied between 100 and 300 mm and the maximum diameter can be varied between 50 and 80 mm.

3. A device according to claim 1 or 2, characterized in that reference signs are provided on the outer surfaces of the internal and external tubes (1, 2) for indicating the correct use of the device.

4. A device according to anyone of the preceding claims, characterized in that the tubes (1, 2) forming the expansion chamber (3) are completely separable.

5. Use of the device according to anyone of the preceding claims, characterized in that the metered aerosol dispenser is housed inside the internal tube (1) of the expansion chamber (3) and the internal tube (1) is in turn stuck into the external tube (2) for storage and transportation of the whole assembly.

## Patentansprüche

1. Eine Einrichtung für die Verabreichung von Medikamenten in der Form von dosierten Aerosolen, umfassend zwei koaxiale Rohre (1, 2), die frei innerhalb einander verschiebbar sind, wobei die Rohre eine innere Expansionskammer (3) begrenzen, deren Volumen entsprechend dem Betrag des Ausziehens des inneren Rohrs (1) relativ zu dem äußeren Rohr (2) verändert werden kann, wobei das äußere Rohr (2) ein offenes Ende (4) zum Einfügen des inneren Rohrs (1) und ein entgegengesetztes Ende (5) hat, und das innere Rohr (1) auch ein offenes Ende (10) hat, das in das äußere Rohr (2) eingefügt ist, und ein entgegengesetztes Ende (11), das mit einem Aerosolausgabegerät verbindbar ist, wobei das entgegengesetzte Ende (5) des äußeren Rohrs (2) verjüngt ist und ein Einwegventil (8) darin untergebracht ist, auf welchem Ventil ein Mundstück (9) angebracht ist, und wobei die Öffnung des Mundstücks (15) des Ausgabegeräts, die Rohre (1, 2) der Expansionskammer (3) und die Öffnung des Mundstücks (9) des letzteren auf der gleichen Achse sind, dadurch **gekennzeichnet,** daß das Ende (11) des inneren Rohrs (1) eine Öffnung (13) hat, die auf der inneren Seite mit einem Satz von Lamellen versehen ist, auf welche Öffnung verschiedene Arten von Mundstücken (15) von konventionellen Ausgabegeräten für dosierte Aerosole aufgesteckt werden können.

2. Eine Einrichtung gemäß Anspruch 1, dadurch **gekennzeichnet,** daß das Volumen der Expansionskammer (3) zwischen etwa 200 und 600 ml variiert sein kann, daß die Gesamtlänge zwischen 100 und 300 mm variiert sein kann, und daß der maximale Durchmesser zwischen 50 und 80 mm variiert sein kann.

3. Eine Einrichtung gemäß Anspruch 1 oder 2, dadurch **gekennzeichnet,** daß Bezugszeichen auf der äußeren Oberfläche des inneren und äußeren Rohrs (1, 2) für das Angeben des korrekten Gebrauchs der Einrichtung vorgesehen sind.

4. Eine Einrichtung gemäß irgendeinem der vorhergehenden Ansprüche, dadurch **gekennzeichnet,** daß die Rohre (1, 2), welche die Expansionskammer (3) bilden, vollständig trennbar sind.

5. Verwendung der Einrichtung gemäß irgendeinem der vorhergehenden Ansprüche, dadurch **gekennzeichnet,** daß das Ausgabegerät für dosiertes Aerosol innerhalb des inneren Rohrs (1) der Expansionskammer (3) untergebracht wird, und das innere Rohr (1) seinerseits in das äußere Rohr (2) für die Aufbewahrung und den Transport der gesamten Anordnung gesteckt wird.

## Revendications

1. Dispositif pour l'administration de médicaments sous forme d'aérosols dosés, comprenant deux tubes coaxiaux (1, 2) coulissant librement l'un dans l'autre, lesdits tubes délimitant une chambre d'aspiration interne (3) dont on peut faire varier le volume en fonction du degré d'extension du tube interne (1) relativement au tube externe (2), ledit tube externe (2) ayant une extrémité ouverte (4) pour l'insertion du tube interne (1) et une extrémité opposée (5), et le tube interne (1) ayant également une extrémité ouverte (10) insérée dans le tube externe (2) et une extrémité opposée (11), connectable à un dispensateur d'aérosols, ladite extrémité opposée (5) du tube externe (2) étant conique et munie d'une valve unidirectionnelle (8), une embouchure (9) étant montée sur ladite valve, et l'orifice de l'embouchure (15) du dispensateur, les tubes (1, 2) de la chambre d'aspiration (3), et l'orifice de l'embouchure (9) de cette dernière se situant sur un même axe, ladite extrémité (11) du tube interne (1) ayant un orifice (13) muni du côté intérieur d'un ensemble de lamelles, orifice sur lequel il est possible d'adapter différents types d'embouchures (15) de dispensateurs classiques d'aérosols dosés.

2. Dispositif selon la revendication 1, caractérisé en ce que le volume de la chambre d'aspiration (3) peut être variée entre environ 200 et 600 ml, la longueur totale peut varier de 100 à 300 mm et le diamètre maximum peut varier de 50 à 80 mm.

3. Dispositif selon la revendication 1 ou 2, caractérisé en ce que des signes de référence sont portés sur les surfaces externes des tubes interne et externe (1, 2) pour indiquer l'utilisation correcte du dispositif.

4. Dispositif selon l'une quelconque des revendications précédentes, caractérisé en ce que les tubes (1, 2) formant la chambre d'aspiration (3) sont complètement séparables.

5. Utilisation du dispositif selon l'une quelconque des revendications précédentes, caractérisé en ce que le dispensateur d'aérosol dosé est logé dans le tube interne (1) de la chambre d'aspiration (3), et le tube interne (1) est à son tour glissé dans le tube externe (2) en vue du stockage et du transport de l'ensemble.
